# EUROPEAN PATENT SPECIFICATION

(11) **EP 2 073 874 B1**
(45) Date of publication and mention of the grant of the patent: **26.01.2011**
(21) Application number: 07823936.5
(22) Date of filing: 26.09.2007
(51) Int. Cl.: A61M 5/32

(54) **NEEDLE SAFETY DEVICE**
NADELSICHERHEITSVORRICHTUNG
DISPOSITIF DE PROTECTION D'AIGUILLE

(30) Priority: 26.09.2006 EP 06254966
(43) Date of publication of application: 01.07.2009
(73) Proprietor: STICK SAFE LIMITED, Stanmore Middlesex HA7 4XR (GB)
(72) Inventor: KORN, Michael, Jonathan, London SW7 2EU (GB)
(74) Representative: Hedley, Nicholas James Matthew
(86) International application number: PCT/GB2007/003673
(87) International publication number: WO 2008/037999

(56) References cited:
- WO-A-01/70595
- US-A1- 5 195 982
- US-A1- 5 279 577
- US-A1- 5 718 689
- US-A1- 5 975 295

## Description

### Technical Field

The present invention relates to a device for assisting in the removal and replacement of the sheath that covers a medical needle, e.g. a hypodermic needle. The term "medical needle" and similar expressions used in the present specification is intended to cover any needle used in medicine, especially those used with a syringe or tubing, whether for injecting liquids into a human or animal patient or for removing materials from the patient, e.g. a catheter needle, and includes needles used during non-therapeutic application, e.g. in laboratory research.

### Background Art

"Needle stick injury" is a common and dangerous problem for all users of medical needles and describes the accidental jabbing of oneself or others with used, contaminated needles, which can lead to health care workers contracting blood-borne diseases, e.g. HIV infection and hepatitis, from the patients they are treating. Needle stick injury is the second most commonly reported accident in the UK National Health Service, with around 100,000 cases reported annually. The conventional method to make a needle safe after use is to re-sheath the used needle, i.e. push it back into its protective plastic sheath. However, it is common for needle stick accidents to occur while a health worker is actually re-sheathing a contaminated needle as it is easy to miss the sheath and stab oneself and so this procedure has been banned by most UK National Health Service Trusts. As such, used needles are carried around hospital wards on open trays and taken to sharps' bins (see below) where they are disposed of. If health workers have a needle stick injury, they must undertake a series of tests to see if they are infected, which is expensive for the health authority. There is also, of course, a substantial cost associated with treatment of infected health workers, litigation, stress and illness related absenteeism and safety equipment and procedures. These problems cost the UK National Health Service millions of pounds per year. This is also a global problem, and is not limited only to health workers but to anyone who uses needles, e.g. diabetes sufferers or their carers.

Currently, by way of an example, when an injection is to be given, an ampoule containing the injectable solution is placed on a small hospital tray together with a medical syringe and needle for delivering the solution; the tray with the solution, syringe and needle is supplied to a nurse or doctor or is prepared by the health workers themselves. The tray is often made of paper-pulp. After use, the tray, the syringe and the needle are all disposed of.

Hospitals generally separate waste into different types, which are disposed of separately. These may be:
1. Non-contaminated (household) waste;
2. Contaminated waste; and
3. Sharp objects, e.g. hypodermic needles.

The cost of dispensing of category 3 (sharp objects) is dramatically higher than that of contaminated waste, which in turn is higher than that of non-contaminated waste. However, it often happens that the waste is not properly segregated and contaminated waste or non-contaminated waste is disposed of in the containers intended for sharp objects. It has been estimated that around 80% of hospital waste placed in the bins for sharp objects should in fact have been placed in bags for contaminated or non-contaminated waste. This leads to the unnecessary expenditure of the cost of disposing of sharp objects. However, the separation of sharp objects from other objects and the re-sheathing of needles often give rise in itself to needle stick injury, which encourages the disposal of non-sharp objects in bins intended for sharp objects.

There exists a need for a device that allows the needle to be re-sheathed without requiring the medical worker to hold the sheath in the process and without requiring the medical worker to place his/her fingers in the vicinity of the point of a needle. Also, there exists a need to separate a medical needle from a syringe or other liquid injecting or removing conduit while minimising the risk of needle stick injury in order to allow the needle and sheath to be disposed of in a container for sharp objects, while allowing the remaining medical equipment used to be disposed of through less expensive channels.

US-5718689 describes a device for storing contaminated medical needles. The device has an opening into which the needle is inserted. A plurality of teeth or flexible elements are located in the opening that cut into the hub of the needle and prevent the needle from sliding back out of the opening. Such a device is expensive to make.

WO01/70595 also describes a device for storing contaminated medical needles. The device includes a container that has several openings; in register with each opening there is a post and one limb of an L-shaped spring clip holds the clip on the post while the other limb presses against the post. A needle, when inserted into the opening, passes between the post and the limb of the clip that presses against the post and so the needle is pressed against the post by the resilience of the clip, thereby gripping the needle. Again such a device is expensive to make.

US-5975295 discloses a device for positioning and holding needle and syringe assemblies or sheaths and includes a planar element having several openings formed by radially-extending slits in the planar element, which forms flaps between the slits. When a sheath is inserted into a hole, the flaps between the slits deform and hold the sheath, thereby allowing the needle to be removed. The gripping action of the flaps on the sheath is however weak and therefore rigid polymers are preferred. If the planar element were to be made out of cheaper material, e.g. paper pulp / moulded fibre or thin plastic, then it would not work effectively. While this device holds the sheath, it has not been designed to allow for the separation of the needle from the syringe, which is essential for correct and safe disposal. Even if the syringe has been separated from the needle, there exists no method to remove the sheathed needle from the holes in the planar element, thereby necessitating disposal of the whole device and preventing the planar element being re-used.

US-5279577 discloses a device for removing the sheath from a medical needle; the device has a base and a plate that is spaced above the base. The plate has a tapering slot in it. A sheathed needle is passed through the wider part of the tapering slot and the tip of the sheath is inserted into an indentation in the base. The sheath is then tilted so that the tip remains in the indentation but the upper part of the sheath moves along the tapering slot until it is jammed against the slot walls. The sheath is then fixed in position and the needle can be removed. Such a device requires awkward manipulation to remove the sheath and the device itself is expensive to make.

US-5195982 describes a device that can be attached to a needle sheath to hold the sheath while the needle is being removed from, or inserted into, the sheath so that the operator's hands are located at a distance from the sheath, thereby reducing the danger of accidental needle stick injury. Such a device requires two hands to manipulate.

### Disclosure of Invention

By exploiting the different fitting mechanisms that exist between, on the one hand, a needle connector, e.g. on a standard syringe and a needle and, on the other hand, between a needle and a needle-protective sheath, a device has been designed which enables one-handed safe use for unsheathing, sheathing and disposal of medical needles. This involves insertion of the syringe into the needle base to attach the needle to the syringe, retraction of the needle from its sheath, use of the needle, insertion of the now contaminated needle back into its sheath, removal of the syringe from the needle by a twisting mechanism and then separation and disposal of the sharps and non-sharps.

The present invention is defined in the accompanying claims.

According to the present invention there is provided a device that is configured to grip the sheath of a medical needle so that the needle can be retracted from it and to hold the sheath while the needle is in use, which device comprises at least two elements that are resiliently connected together and at least partially define at least one opening into which the sheath can be pushed. The at least two elements and the at least one opening act as a valve that allows the sheath to be pushed into the opening along a first direction but grips the sheath if the sheath is pulled out of the opening in a second direction that is opposite to the first direction. In other words, the force needed to pull the sheath out of the device in the second direction is greater than the force needed to push the sheath into the device in the first direction. The device, when holding the sheath, will also prevent the sheath from twisting, to allow the syringe to be separated from the needle after use by pushing the needle into the sheath held by the device and then applying a twisting movement with a retraction in the second direction to detach the needle from the syringe.

By gripping the sheath, the device allows the medical needle to be retracted from the sheath and pushed back into the sheath while keeping the medical worker's hands away from the needle point. This minimises the danger of needle stick injury. The device can be operated by a health worker (or other user) as follows: attaching the sheathed needle to the syringe, holding the syringe to which the needle and sheath are attached, pushing the sheath into the opening and pulling the syringe in the opposite direction, which causes the sheath to be gripped by the device, and allows the needle to be pulled out of the sheath, which is held by the device. Also, the sheathed needle can be put into the device first, and then the syringe can be pushed into it and then retracted from the sheath. After use, the needle is pushed into the sheath that is held by the device and the syringe is detached by a twisting and pulling movement. The sheathed needle can optionally be removed from the device since it is safe to handle in its sheathed condition and disposed of in the appropriate way; alternatively, the device and the sheathed needle may be disposed of together, which means that it is not necessary for the sheathed needle to be removed from the device. The device can be held by the user at a location spaced away from the opening, i.e. the user's fingers are located away from the opening, so that the danger of needle stick is reduced.

In a first embodiment, the device includes two elements that are resiliently connected together by a hinge. Each element includes a hole of a size to accommodate the sheath snugly. The two elements are not parallel to each other so that the holes are not directly aligned with each other. When the sheath is pushed into the holes of the two elements, the elements flex with respect to each other about the resilient hinge, thereby bringing the holes closer into alignment and allowing the sheath to be pushed into the holes. When holding the sheath after it has been pushed in and the pushing force released, the resilience of the hinge and/or the action of trying to retract the sheath from the holes urges the hinge to flex back, i.e. so that the holes are less in alignment, which increases the gripping force on the sheath. Thus a force exerted trying to remove the sheath is resisted because the engagement between the sheath and the edges of the holes would move the elements apart, which would take the holes further out of alignment and so increases the jamming action of the sheath in the holes. This means that the elements grip the sheath and resist it from being removed from the device when the sheath is held in the device.

The sheath is usually held on the needle by friction or a push fit (also called a "pop" or "snap" fit), the retraction of the needle from the sheath can be effected by a simple pulling action. Thus, when a syringe with a sheathed needle is held by the device, the action of pulling the syringe increases the gripping force of the device on the sheath and so allows the needle to be unsheathed without pulling the sheath out of the device.

The first embodiment is not limited only to two elements but further elements, each with holes or openings and each resiliently attached to a preceding element, can be used. In this way, the sheath can be gripped by three or more elements in the manner described above.

The snug fit between the sheath and the holes in the elements may be provided by round or square holes, whose rim engages with, and preferably is deformed by, the insertion of the sheath. Alternatively the holes may include one or more secondary elements that are resiliently connected to the area surrounding the hole and are pushed back when the sheath is pushed into the hole but their resilience grips the sheath in the hole.

In a second embodiment, the elements may be resiliently connected together and form an opening between them. In this case, the elements are angled towards each other and extend partly in the direction that the sheath is inserted into the opening so that the sheath can be pushed in one direction (the insertion direction), which tends to expand the opening and thereby allows the sheath to pass through. However, because of the angle between the elements, when the sheath is pulled in the opposite direction (opposite to the insertion direction), the engagement of the sheath with the elements tends to close the opening and resists the sheath being removed from the device. The elements may be integrally formed, e.g. in a single moulding. The elements are part of a structure that extends in the direction of insertion of the sheath such that the structure can be compressed to align the elements with an inserted sheath to allow the sheath to be withdrawn from between the elements. Conveniently, the elements can form part of a dome such that the sheath can be pushed through the concave face of the dome while the elements resist the sheath moving in the opposite direction.

In the first embodiment described above, it is stated that the holes in the elements may themselves have secondary elements; these secondary elements may be angled towards each other and extend partly in the insertion direction, as described in connection with the second embodiment.

The gripping of the sheath by the device not only allows the needle to be retracted from the sheath but also allows the needle, after use, to be reinserted into the sheath with one hand since the sheath is held in a stable position, thereby reducing the instances of needle stick injury. In addition, the gripping action on the sheath, which is especially strong when the user pulls a sheathed needle in the retraction direction, allows the syringe to be twisted and so disconnects the needle from the syringe, thereby allowing the syringe to be disposed of separately from the needle using a cheaper disposal method than that used for sharps.

In this specification, the invention is often described by reference to a "syringe", but it should be borne in mind that the invention is equally applicable to other elements that are attached to a needle, e.g. tubing that allows material to be inserted into or withdrawn from a patient or other person, for example a needle attached to tubing that is used to withdraw blood from a blood donor.

The needle does not have to be connected to any other element; for example a needle may be used to insert a cannula into the body; a cannula is a flexible tube that, when inserted into the body, is used either to withdraw fluid or to insert medication. In order to insert a cannula into a patient, a needle is pushed into the bore of the tube and the cannula with the inserted needle can penetrate into the body; after insertion, the needle is removed leaving the tip of the cannula in place. A cannula needle does not have a self-fitting sheath like hypodermic needles have, and is therefore usually placed inside a tray or sometimes speared into the paper pulp material that a hospital tray is made of; even when speared into the tray material, the needle can easily be knocked to expose the pointed end of the needle. In either case, the needle point is not adequately isolated and this gives rise to needle stick injury. In a variant of the first embodiment, the needle can be protected by a device that comprises at least two elements that are connected together by a hinge. The elements each include an area that can be penetrated by the cannula needle, e.g. an area that is softer, i.e. more easily penetrated, or thinner than the rest of the element. The holes formed by the needle in the penetration areas are the same size as the needle and so hold the needle snugly. If an attempt were made to retract the needle by pulling it in a direction that is opposite to the direction of insertion, the friction between the needle and the elements tend to move the elements apart, thereby taking the holes out of alignment and so gripping the needle tighter. This will hold the cannula needle until it can be disposed of safely. This mechanism is not limited to cannula needles but it finds greatest application with cannula needles since they do not have a protective sheath into which they can be inserted to provide protection against needle stick injury.

According to a further aspect of the present invention, the device may be incorporated into a holder, e.g. a hospital tray, that is capable of holding a medical needle, a sheath fitted over the needle and a syringe. The device may be separable from the rest of the holder, e.g. by means of a line of weakness or by being temporarily secured to the tray. This allows a re-sheathed needle held in the device to be separated from the syringe; the device and the re-sheathed needle can then be separated from the holder and disposed of in a suitable container for sharp material, while allowing the holder and the syringe to be disposed of correctly and therefore cost and environmentally effectively. Alternatively the sheathed needle may be removed from the device. In the case of the first embodiment, this may be achieved by squeezing the two elements together so that the holes are brought closer into alignment and this releases part of the gripping force on the sheath, allowing it to be easily removed from the device. In the case of the second embodiment, the elements holding the sheath are part of a structure made of compliant material that extends in the direction of insertion of the sheath such that the structure can be compressed to align the elements with an inserted sheath to allow the sheath to be withdrawn from between the elements.

The device may be integrally formed with the holder, for example if the holder is a tray made from paper-pulp, the two elements of the device in the first embodiment may be two flaps of paper-pulp attached to the rim of the tray. Because the device can be integral with the rest of the holder, its inclusion need not increase the cost of the holder significantly.

Similar considerations apply if the holder were made of plastic materials or other disposable or reusable materials. It is possible to make some of the embodiments of the present invention out of metal.

### Brief Description of the Drawings

There will now be described, by way of example only, several designs of the device in accordance with the present invention by reference to the accompanying drawings in which:
Figure 1 is a schematic exploded view of a syringe, needle and sheath or sheath of standard design;
Figure 2 is schematic view of the steps for attaching the hypodermic needle to a syringe, unsheathing the needle, re-sheathing the needle, removing the syringe and disposing of the used needle using a device in accordance with a first embodiment of the present invention;
Figure 3 is a sectional view through part of the first design of the device of the present invention both before it holds a syringe sheath (Figure a) and while it is holding a sheath (Figure b); Figure 3(c) is a plan view of the fingers 27;
Figure 4 is a sectional view through part of a second design of the device of the present invention showing the device before holding a syringe sheath (Figure a) and while holding a syringe sheath (Figure b);
Figure 5 is a sectional view through part of a third design of the device of the present invention showing the device before holding a needle sheath (Figure 5(a)) and while holding a needle sheath (Figure 5(b)) and how when a sideways force D is applied, it is easy to retract the sheath.
Figure 6 is a sectional view through part of a fourth design of the device of the present invention showing the device before holding a needle sheath (Figure 6(a)) and while holding a needle sheath (Figure 6(b));
Figure 7 is a sectional view through part of a design of the device of the present invention incorporated into a tray, showing the device holding a syringe and sheathed needle (Figure a), the tray before use showing its stackability (Figure b), the device holding a sheath (Figure c); and a variant on the tray (Figure d);
Figure 8 is a series of perspective views of a further tray incorporating a device of the present invention, showing the device in various stages of handling a syringe and needle;
Figure 9 is formed of a series of perspective views of another tray incorporating a device of the present invention, showing the device in various stages of handling a syringe and needle;
Figure 10 is a perspective view of a cannula needle; and
Figure 11 is a sectional view of a device of the present invention for holding a cannula needle.

### Best Mode for Putting Invention Into Operation

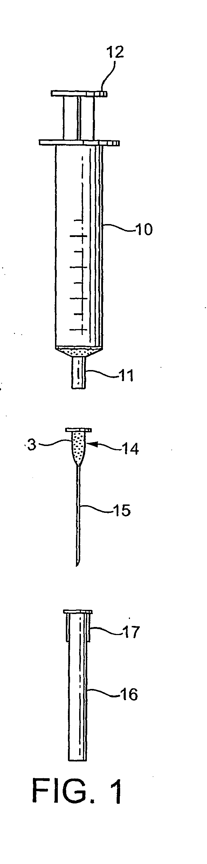

Referring initially to Figure 1, there is shown a syringe 10, needle 14 and sheath 16 of standard design; the same sized sheath is generally used for all needle sizes, although canula needles and vacutainer needles have different sized sheaths. The device of the present invention, if suitably configured, can accommodate a range of different sizes of sheath. The cross section dimensions of standard sheaths is a 4mm square, although at least one sheath manufacturer makes sheaths that are 5mm square. Certain specialised needles, e.g. sheaths for vacutainer needles go up to 10mm.

The syringe 10 has a plunger 12 that can be moved downwardly to deliver injectable material through a cylindrical outlet 11. The hypodermic needle 14 includes a hollow needle 15 and a collar 13 that can be pushed onto the syringe outlet 11 and be held there by friction. The frictional engagement can be broken by twisting the syringe 10 relative to the needle 14 and applying a slight pulling away force.

The needle 15 is protected by a plastic sheath or cap 16 that can be friction fitted with a 'pop' fit onto the collar 13 of the needle 14. The engagement is such that the needle cannot rotate readily within the sheath 16. As such, the needle and sheath can be removed as a unit from the syringe 10 by holding the sheath and applying a twisting and pulling motion to the syringe. Ridges 17 are provided on the outside of the sheath or the sheath is of non-circular (e.g. square cross section) to assist in gripping the sheath. In contrast to the twisting motion used to remove the needle/sheath unit as a whole, the sheath 16 can only be removed from the needle by pulling the sheath with a force sufficient to overcome both the friction between the sheath and the needle and also the "pop" fitting. The pop fitting is a combination of at least one projection on either the needle or the sheath that engages with at least one recess in the other. Since the projections and/or recesses are made of deformable material, the pop fitting can be overcome by pulling the sheath with a force necessary to deform the fitting and allow the projection(s) to be removed from the recess(es).

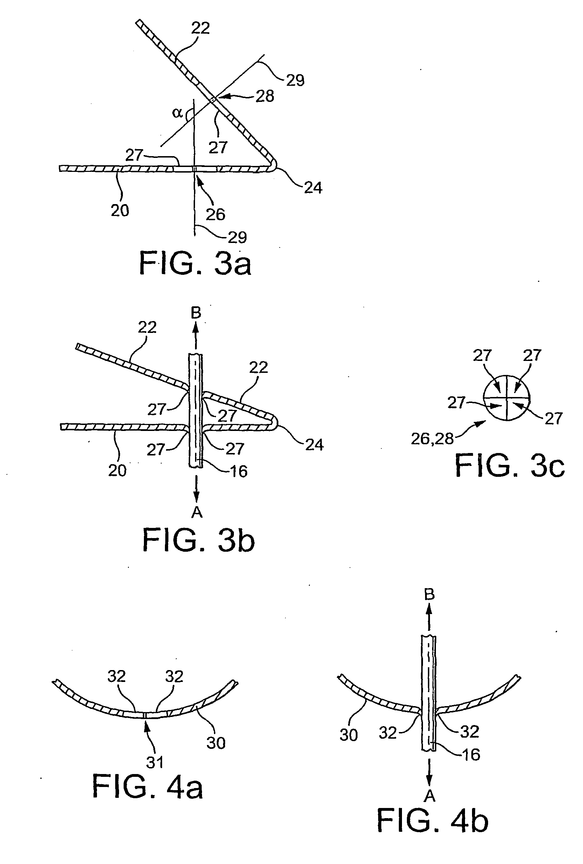

Referring now to Figure 3, there is shown a device in accordance with the present invention, which includes two elements 20, 22 that are connected by a resilient hinge 24. The device can be made simply by folding a sheet along the hinge 24 to make the two elements. This folded hinge may be curved to provide extra stiffness. The device may therefore be made out of any material that is stiff and has resilience, including paper-pulp (often known as moulded fibre) material, plastic material or metal.

Punched into the elements 20, 22 are two openings 26, 28 formed by cross shapes; alternatively square cut holes, or round holes may be provided or indeed any shape of hole that provides a snug fit for the sheath within the hole; such a snug fit may be caused by the edge of a hole being deformed by the insertion of the sheath.

Each cross shape forms four resilient fingers 27 in each element that can be pushed out by a sheath 16 of a hypodermic needle, as shown in Figure 3(b), which shows the sheath 16 once it has been pushed in the direction of arrow A through the openings 26, 28; in the process, the movement of the sheath into the openings causes the fingers 27 of the openings to bend downwardly in the direction of arrow A. The bent fingers 27 provide a snug fit and prevent the sheath being pulled in the direction B (opposite to the direction of arrow A) because, if the sheath is pulled in this direction, the frictional engagement between the sheath and the fingers 27 will tend to close the openings 26, 28 and thereby make the removal of the sheath in this direction harder. The fingers could also prevent the sheath from being turned within the holes 26, 28, similarly if the hole was a close square fit or a tight hole, the sheath would not be able to rotate.

When the device is deployed ready to receive a needle sheath, the openings 26, 28 are not aligned with each other, i.e. the axes 29 of the two openings are out of alignment and form an angle a of 170 to 110°, and so the insertion of the sheath 16 into the opening 28, causes the top element 22 to flex about the hinge 24, which brings the openings closer into alignment. The sheath is then pushed further into the opening 26 in the lower element 20. The resilience of the fingers 27 causes them to press against the sheath, which holds the sheath tightly in the openings. When an attempt is made to move the sheath 16 in the direction of the arrow B, the tight engagement of the sheath in the opening 28 causes the upper element to move away from the lower element about the hinge 24, but this brings the two elements further out of alignment which makes the engagement of the sheath in the openings tighter, thereby resisting the sheath from being moved in the direction of arrow B.

Generally, it has been found that two or more elements will be required to hold the sheath in place but if the force exerted by the fingers 27 is large, then it may be possible to use only one element. However, the use of only one element is assisted if the fingers extend partly in the sheath insertion direction A, e.g. the fingers together form a domed element, as shown in Figure 4. The element 30 in Figure 4(a) is domed; the curvature may be in only one plane i.e. the element 30 may form parts of the wall of the cylinder, or the curvature may be applied in two directions, e.g. the element 30 may be a section of a three dimensional dome. A cross shape 32 (as shown in Figure 3(c)) or a square or round hole is applied in the element 30 to form the opening 31 and the sheath 16 may be pushed from the concave side of the dome through the opening 31 in the direction of the arrow A, thereby deforming fingers 32 of the opening. As described above, the fingers 32 will resist the movement of the sheath in a direction opposite to that of insertion, i.e. it resists motion in the direction of the arrow B. The dome shape of the element 30 adds extra strength to the element and prevents it from distorting when an attempt is made to pull the sheath 16 in the direction of the arrow B. The additional resistance to the removal of the sheath brought about by the rounded or angled shape of the element 30 means that it is more feasible to use only one element in the present arrangement, in comparison to the two elements provided in the arrangement of Figure 3.

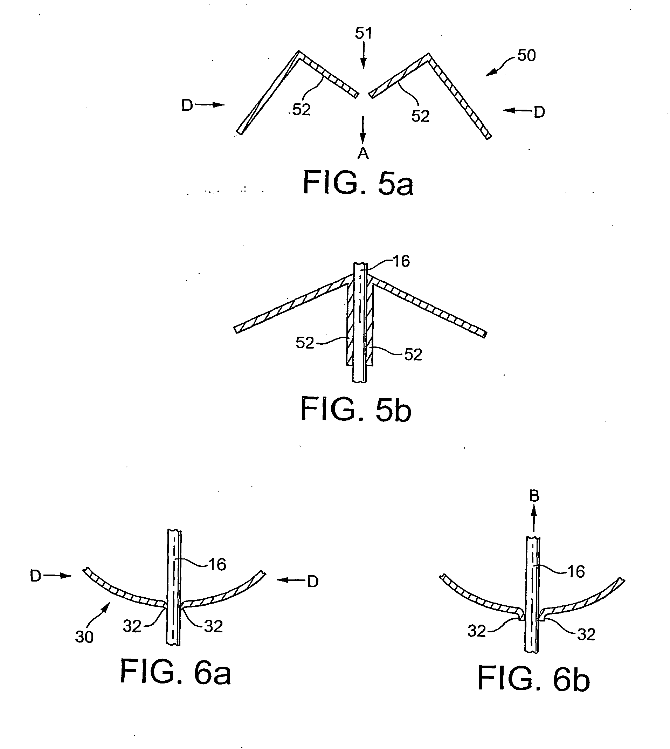

The dome 30 also allows for removal of the sheath from the openings by squeezing the dome 30 in the directions of arrows D (see Figure 6(a)), which causes the fingers 32 to align with the sheath walls (see Figures 6(b)), thereby allowing the sheath to be withdrawn in the direction of the arrow B.

The domed shape arrangement of the fingers can be used to form the openings 26,28 in the hinged elements 20,22 shown in Figures 3a and 3b instead of the planar configuration shown in Figure 3c.

Alternatively, the element shape could be 'M' shaped in cross section, as shown for element 50 in Figure 5. In Figure 5, the element 50 has a central 'V' shape with a hole 51 in the bottom such that the side walls 52 act as fingers and bend in to allow insertion of the sheath in the direction of the arrow A but the V will prevent retraction of the sheath as the side walls are forced out further as the bottom of the V is lifted up. As shown in Figure 5(b), when the two parts of the 'M' shape are squeezed together (see arrows D), the sidewalls 52 are aligned with the sheath 16, allowing the sheath to be retracted in the upward direction (as seen in Figure 5(b)).

Any number of fingers 27, 33 may be provided in the openings 26, 28, 31 and 51 although preferably 2 to 6 fingers are provided. However, 4 fingers are preferred since they fit in with the 4 ridges / sides 17 on the sheath, and thus prevent the sheath from rotating in the opening; if there were not the same number of slots as ridges, the grip strength would be reduced.

The use of the fingers 27, 32 allows for a wide variety of different diameter sheaths to be opened using the arrangement of the present invention.

More than two elements 20, 22 may be provided each connected to the preceding element by a resilient hinge 24.

The holes or openings in the device of the present invention will generally be shaped and sized to accommodate sheaths having a 4-6 mm square cross section snugly, although holes to accommodate sheath cross sections up to 10mm square may be needed.

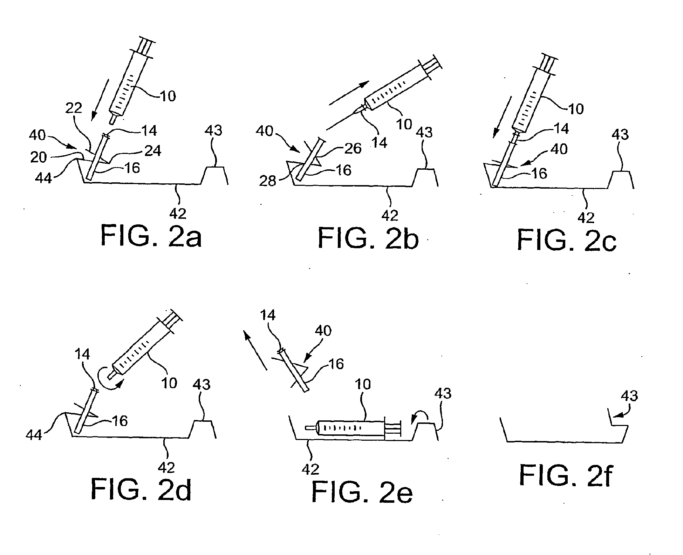

Turning to Figure 2, there is shown, in cross section, a tray 42 which may be made out of shaped paper-pulp or any other suitable material for forming hospital trays.

A sheath-retaining device 40 in accordance with the present invention is attached along one edge of the tray 42. The device 40 and the tray 42 may be formed integrally with each other, in which case the addition of the device 40 only increases the manufacturing cost of the tray very slightly. The device may be joined to the tray by a line of weakness 44. Alternatively, the device could be made separately and removably attached to the tray 42, e.g. by means of a 'slot fit', or it may be integrated into the tray.

The syringe 10 and the sheathed needle 14, 16 may be provided as separate items in the tray. The needle in its sheath is pushed into the device of the present invention, as described in connection with Figure 3, which holds it in position. The needle collar 13 is then pushed onto the syringe outlet 11 such that the collar 13 is held on the outlet 11 by friction.

By either holding the tray 42 in one hand and pulling on the syringe 10 with the other hand, or resting the tray on a surface and pushing the wrist of one hand on the wrist rest 43 and pulling the syringe with the same hand, a medical practitioner can unsheath the needle 14 (see Figure (b)), which can be put into operation in the normal manner. The use of the wrist rest 43 holds the tray and prevents it from moving or tipping and has the advantage that the whole procedure can be performed with only one hand leaving the other hand free. Because the user's hand is nowhere near the needle when the needle is pushed onto the syringe, needle stick injury can be avoided if the needle accidentally becomes unsheathed.

After completion of its normal operation, the needle 14 is re-sheathed by pushing it into the sheath 16 (see Figure 2(c)). In this operation, the sheath 16 can engage at the bottom edge of the tray to prevent it being pushed through the device 40. It is important to note that the user can hold the tray with one hand in a position well away from the sheath and the needle 14 and accordingly the risk of needle stick injury in these circumstances is vastly reduced. Alternatively, the user can press down on the wrist rest 43 of the tray with the wrist of the hand which is holding the needle and insert the needle into the sheath. The tray has been designed to have a wide-stable base, with extra support provided by the wrist support 43 at the back. The rim of the tray could be extended downwardly so that it is level with the base of the tray to provide additional stability. As such, the operation can be comfortably completed using only one hand. This is the safest method, as it eliminates the possibility of stabbing the other hand which can be kept well away, or the other hand can be used to do another operation such as comfort the patient. The sides of the tray may be formed into handles to facilitate the tray being carried at locations away from the device to avoid the possibility of needle stick by the user's hands being close to the device. The handles will be described in further detail below.

The device depicted further reduces the risk of needle stick injury as the contaminated needle is 'made safe' immediately after use by safely sheathing it. Currently because of the discouragement of, or ban on, the practice of re-sheathing needles manually, the exposed needles are often kept on their bulky cumbersome syringes which rest in the shallow trays, and are carried across the ward to the sharps bin where they are finally separated from the syringe and disposed of. Often the syringe and the needle will wrongly be placed into the sharps bin together. This practice is costly and dangerous, as the bins fill up quickly and can easily become overfilled with exposed needles poking out through the opening. Many needle stick injuries occur at the bin for this reason. This method would eliminate that, as not only is the needle disposed of separately from the syringe, but also the needle is disposed within its sheath, making it doubly safe.

Because the sheath 16 is held and prevented from rotating in the device 40 of the present invention, the syringe 10 can be removed from the needle by a simple twisting action (see Figure 2(d)) leaving the sheathed needle 14, 16 held by the device 40 while the syringe 10 has been separated and can be placed in the tray 42. The sheathed needle can be removed manually from the device 40 or, alternatively, the device 40 may be removed along the line of weakness 44 and disposed of together with the sheathed needle held by the device. The advantage of removing the device 40 and the sheathed needle from the rest of the tray is that it can be disposed of in the bin designated for sharp articles, while allowing the tray 42 and the syringe to be disposed of more cheaply and appropriately.

The manual removal of the sheathed needle from the device 40 can be achieved by squeezing the elements 20,22 towards each other, thereby bringing the holes 26,28 into closer alignment and reducing the gripping force of the elements 20,22 on the sheath, thereby allowing the sheath to be retracted.

The wrist wrest 43 as described earlier can be folded over into the tray 40 as shown by arrow C in Figure 2(e) (see Figure 2(f)) and acts as a thumb guard when carrying the tray, thereby preventing the thumb from coming into contact with the contents of the tray. This will further protect the health worker from potential injuries and contamination from other hazardous materials and equipment which may be in the tray. The wrist support may be elongated so that it is approximately the same length as the tray (not shown) so that it is able to be folded over and cover the well of the tray and prevent the contents spilling out; a latch may be provided to keep the folded over cover in place.

The tray used in connection with the present invention may be deeper than many of the current trays on the market, making them better designed for hospital ward use. The wrist support 43 has a wide base which provides extra stability. The shape and design of the tray is non intrusive and pleasing to the eye, further improving on the fact that this is also a patient friendly product. The tray can be designed to be stackable.

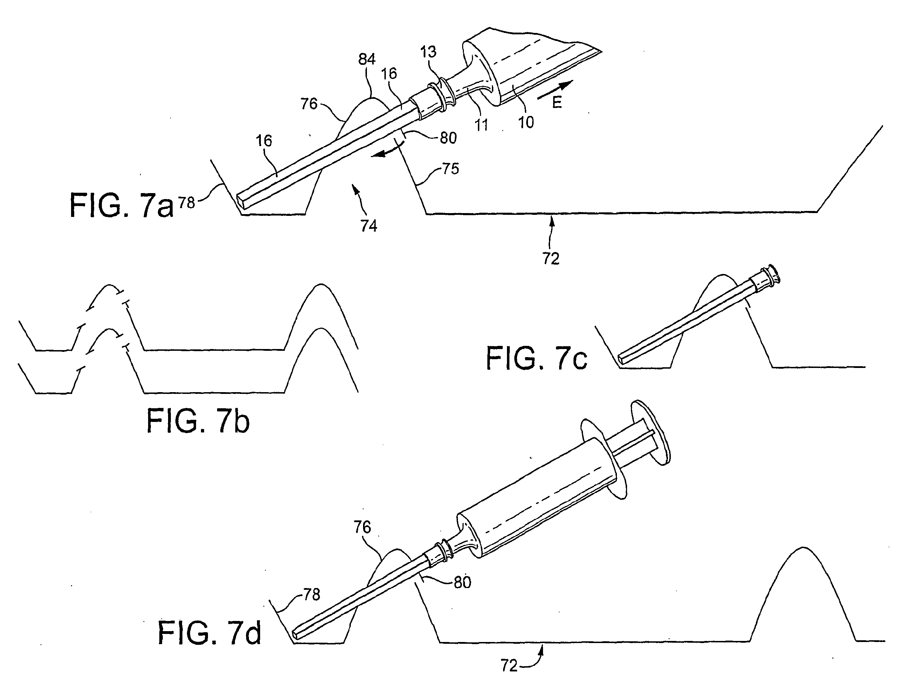

Figure 7 shows a further design of tray 72 that includes an integral device 74 of the present invention. Figure 7a shows the tray in cross section and the device 74 is integrated in one end of the tray; the end of the tray has three sections - an inner wall 75, an outer wall 76 and a stop wall 78. A tab 80 is cut out of part of the inner wall and has a hole therein that can accommodate the sheath (as shown) snugly. Likewise the outer wall also has a hole therein that can accommodate the sheath (as shown) snugly. The inner and outer walls are connected together by a hinge 84 formed by the ridge between the walls and the walls (and in particular the tab 80 and the outer wall 76) can flex about the hinge towards and away from each other. As can be seen, the tab 80 and the outer wall 76 together act in the same way as the two elements described in connection with Figures 2 and 3. The end stop wall 78 limits the extent that the sheath 16 can be pushed through the tab and the outer wall. As with the Figure 2 and 3 arrangement, the action of pulling the syringe 10 in the direction of the arrow E brings the holes in the tab 80 and the outer wall 76 further out of alignment, which increases the gripping force of the engagement of the sheath in the device, thereby enabling the sheath to be pulled off the needle, with the sheath 16 being held in the device (see Figure 7c). The needle can be returned to the sheath after use. The needle can be disconnected from the syringe by pulling and simultaneously twisting the syringe; because the sheath is held in the device and the needle cannot turn within the sheath, the friction or screw thread connection between the needle collar 13 and the syringe outlet 11 is released and the syringe 10 is thereby separated from the needle. The sheathed needle can be removed from the device 74 by pressing the tab 80 in the direction of the arrow F, which brings the holes in the tab and the outer wall into alignment (or keeps the holes in alignment) and so reduces the grip on the sheath and allows it to be pulled out from the device.

The advantage of the tray of Figure 7 is that is stackable (see Figure 7b).

The angles of the tray of Figure 7a can be rounded (see Figure 7d) and the walls made to slope, which eases manufacture by allowing easy removal of mould parts used in moulding the tray to shape.

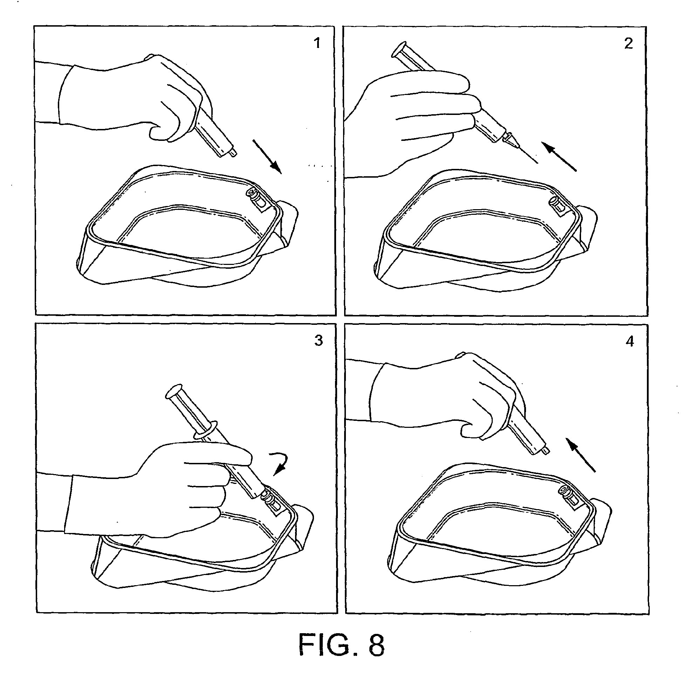

Figure 8 shows a further design of tray with an integral device according to the present invention. The tray is similar in construction to that of Figure 7 and has a tab 80 pressed out of inner tray wall 75, an outer tray wall 76 and an end stop wall 78. The sides of the tray are provided with handles 86 for carrying the tray. As before, holes are provided in the tab 80 and the outer wall 76 to snugly hold the sheath 16.

The operation of the tray is as described before - the needle held in sheath 16 is pushed into the holes in the tab 80 and outer wall 76 and the syringe 10 is pushed onto the needle (Figure 8.1); the syringe 10 is retracted and the device grips the sheath thereby allowing the needle to be unsheathed (Figure 8.2); after use, the needle is returned to the sheath and the syringe is twisted and pulled back to release the needle from the syringe (Figure 8.3 and 8.4).

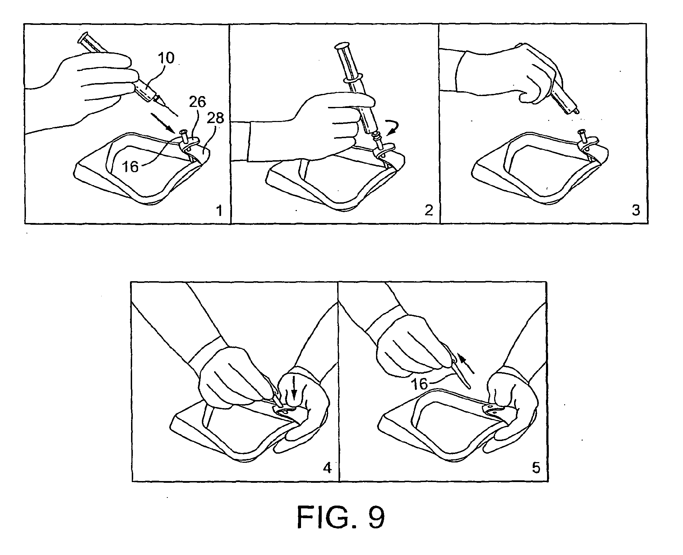

A further tray design is shown in Figure 9 where the device is as shown and described in connection with Figures 2 and 3. The stages of use shown in Figures 9.1 to 9.3 are the same as those shown in Figures 2c and 2d. Instead of tearing off the device (as in Figure 2e), the sheathed needle is removed from the device by pressing on the element 26 to bring the holes in the elements 26,28 into closer alignment (Figure 9.4) and so reducing the grip on sheath and allowing it to be pulled out from the device (Figure 9.5).

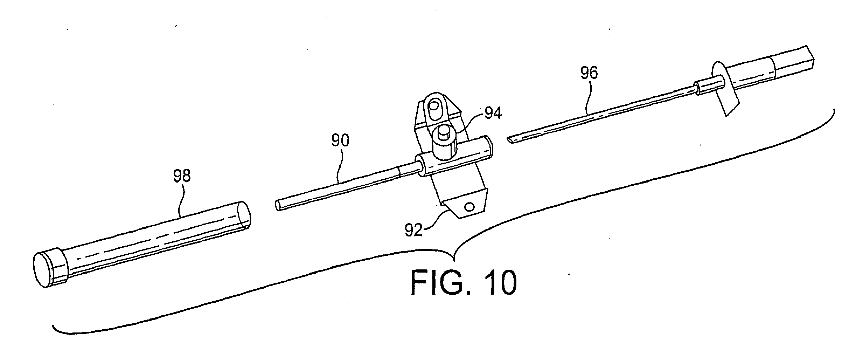

Figure 10 shows a standard cannula, which consists of a flexible hollow plastic tube 90 that is inserted into the patient, a cap 94 that allows tubing to be connected to the cannula and wings 92 for securing the cannula in place, e.g. using sticking plaster, and preventing it from falling out of the patient. A metallic needle 96 can be passed through the cap into the bore of the cannula needle to provide rigidity and strength to the cannula while it is being inserted into the patient. Once the cannula has been inserted, the needle is removed and tubing attached to the cap to pass fluids into or out of the patient.

The cannula is supplied with the needle inserted into the cannula bore and the assembly is protected by a sheath, which engages the cannula cap 94 to keep it in place. Once the cannula has been inserted and the needle 96 is removed, the needle cannot be inserted back into the sheath to protect the user from needle stick injury because the sheath is designed to engage the cannula cap 94 and not the needle, which therefore has to be placed in a safe place, but the presence of an unsheathed needle, even in a seemingly safe place, is hazardous.

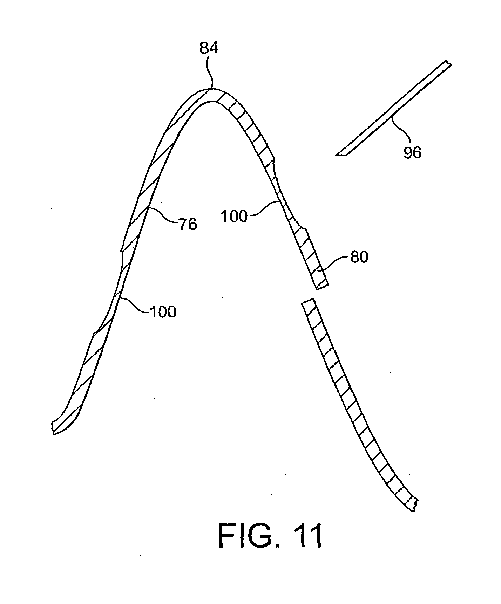

The needle can be secured in a safe condition as shown in Figure 11. The arrangement in Figure 11 is similar to that of Figure 7 except that instead of having pre-formed holes in the tab 80 and the outer wall 76, regions 100 with thinned walls are provided; the regions can readily be penetrated by the needle 96. Because the needle itself has made the holes in the tab and the outer walls, there is a snug fit between the device and the needle such that, if a force were exerted on the needle to remove it, the inner and outer walls would flex around the hinge 84, causing tighter gripping of the needle. The needle can be removed by holding or pressing the tab to prevent this hinging movement and so allow the needle to be withdrawn. A backstop wall (not shown) is provided in the same way as in Figure 7. A thickened region may be provided on the backstop wall to receive the point of the needle.

Instead of the walls being thinned in regions 100, other arrangements for making the regions easier to penetrate that the rest of the tray may used, e.g. making the regions out of a softer material.

## Claims

1. A device that is configured to grip the sheath (16) of a medical needle so that the needle (15) can be retracted from it with one hand and to hold the sheath while the needle (15) is in use, which device comprises:
a first element (20) having a first opening (26) therein and
a second element (22) resiliently joined to the first element (20) by a hinge (24)
and having a second opening (28) therein,
**characterised in that** the first and second elements (20,22) are out of alignment with each other whereby said first and second elements (20,22) and the first and second openings (26,28) act as a valve so that, when a sheath (16) is pushed into the first and second openings (26,28) and engages the first opening (26), the first element (20) can flex relative to the second element (22) about the hinge (24) and when the sheath (16) is no longer pushed, the resilient connection of the elements (20,22) by the hinge (24) causes the first and second openings (26,28) to grip the sheath (16) and to hold the sheath (16) against rotating within the openings (26,28),

2. A device as claimed in claim 1, wherein the arrangement is such that the action of pulling the sheath (16) out of the openings causes the first and second elements (20,22) to flex relative to each other about the hinge (24), thereby increasing the grip of the elements on the sheath (16).

3. A device as claimed in claim 1 or claim 2, wherein the first and second openings (26,28) are configured to receive a sheath (16) of a needle (15) having a cross sectional width in the range of from 4mm to1 10mm square, e.g. 4 to 6mm square, such that it fits snugly in both the first and second openings (26,28) and optionally the openings are configured to receiving a sheath (16) of a needle snugly that has a cross sectional width anywhere in the range of from 4mm to 10mm, e.g. anywhere in the range of from 4 to 6mm.

4. A device as claimed in any one of claims 1 to 3, wherein, when deployed to receive the sheath (16), notional lines passing orthogonally through the centres of the first and second openings (26,28) lie at an angle α of 170° to 110° to each other.

5. A device as claimed in any one of claims 1 to 4, wherein, when deployed to receive the sheath (16) and the hinge (24) is unflexed, the first element (20) lies at an angle of 10° to 60° to the second element.

6. A device as claimed in any of claims 1 to 5, which includes at least one further element that has an opening therein and that is resiliently connected to a preceding element by a hinge (24), and wherein the opening in each further element lies out of alignment with the opening in the preceding element.

7. A device as claimed in any of claims 1 to 6 made of paper-pulp / moulded fibre, metal or plastic material.

8. A device as claimed in any one of claims 1 to 7, wherein each of the openings (26,28) includes at least one resilient flap (27) that extends into the opening and engages a sheath (16) inserted into the opening to provide a snug engagement of the sheath (16) with the opening.

9. A device that is configured to grip a medical needle so that the needle (15) can be held in a safe condition, which device comprises:
a first element (20) having a first region that a needle (15) can penetrate more easily than the rest of the first element (20) and
a second element (22) resiliently joined to the first element (20) by a hinge (24) and having a second region that a needle (15) can penetrate more easily that the rest of the second element,
**characterised in that** the arrangement is such that, when a needle (15) has penetrated the regions in the first and second elements (20,22) such that the needle (15) is held within holes in the said regions of the first and second elements (20,22), the action of trying to withdraw the needle (15) from the first and second elements (20,22) causes the elements to move about the hinge (24) so that the holes through the first and second elements (20,22) are brought out of alignment, thereby causing the needle (15) to be gripped by its engagement with the first and second elements (20,22)..

10. A device as claimed in claim 9, wherein the said regions are formed of a softer and/or thinner material than the remainder of the respective elements.

11. A device made of compliant resilient material, e.g. paper-pulp or plastic material that is configured to grip the sheath (16) of a medical needle (15) so that the needle (15) can be retracted from it with one hand and to hold the sheath (16) while the needle (15) is in use, which device comprises at least two elements resiliently connected together and an opening formed between the elements into which the sheath (16) can be inserted,
said at least two elements (32,52) and the at least one opening acting as a valve that allows the sheath (16) to be pushed to insert it into the at least one opening but grips the sheath (16) if the sheath (16) is pulled out of the opening, wherein the resilient connection between the elements is configured to hold a sheath (16) against rotating within the opening,
wherein the elements are angled with respect to each other in a configuration such that a sheath (16) when moved in one direction through the opening engages the elements bordering the opening and further movement in that direction tends to expand the opening, thereby permitting the sheath (16) to move further in that direction, and such that a sheath (16) when moved in the opposite direction through the opening engages the elements bordering the opening and further movement in that direction tends to contract the opening, thereby resisting the sheath (16) moving in that direction
**characterised in that** the elements are part of a structure (30,50) that extends in the direction of insertion of the sheath (16) such that the structure can be compressed to align the elements with an inserted sheath (16) to allow the sheath (16) to be withdrawn from between the elements.

12. A device as claimed in claim 11, wherein the elements (32,52) that are angled with respect to each other from part of a dome or "V" shape.

13. A device as claimed in any preceding claim wherein the elements (32,52) are integrally formed, e.g. in a single moulding.

14. A holder, e.g. a tray, for holding a medical needle (15), a sheath (16) fitted over the needle and a syringe, which holder comprises a device as claimed in any one of claims 1 to 13, said device:
optionally being separable from the rest of the holder, e.g. it is joined to the rest of the holder by a line of weakness, or is a separate piece which is connected to the holder and can removed and
optionally including a flat surface on the top of the holder opposite the location of the device that can act as a wrist rest for keeping the holder still while sheathing a needle, which wrist rest can optionally be bent over the holder to provide a thumbguard or a cover for the holder.

15. A method of removing a sheath (16) from a medical needle (15) using a device as claimed in any one of claims 1 to 8, which method comprises:
a. moving the sheath (16) into the openings in the first and second elements (20,22) in a first direction, the sheath (16) optionally fitting snugly into the openings in the first and second elements (20,22);
b. pushing the sheath (16) into the openings to engage the elements bordering the openings;
c. releasing the pushing force to allow the elements to grip the sheath (16);
d. retracting the medical needle from the gripped sheath (16), e.g. by pulling the medical needle in the direction opposite to the first direction, which optionally causes the first and second elements (20,22) to move about the hinge (24), thus increasing the force gripping the sheath (16) until the needle is pulled off the sheath (16);
e. reinserting the needle into the sheath (16) held by the device, and
f. twisting and pulling a syringe connected to the needle to disconnect the syringe from the needle
and optionally:
g. holding the first and/or second element (22) in place to prevent the relative movement of the two elements about the hinge (24), thereby enabling the sheath (16) to be retracted from the device.

## Patentansprüche

1. Vorrichtung, welche zum Erfassen der Hülle (16) einer medizinischen Nadel konfiguriert ist, so dass die Nadel (15) mit einer Hand daraus zurückgezogen werden kann, und zum Halten der Hülle, während die Nadel (15) in Verwendung ist, welche Vorrichtung aufweist:
ein erstes Element (20), das eine erste darin befindliche Öffnung (26) hat, und
ein zweites Element (22), das mit dem ersten Element (20) durch ein Gelenk (24) federnd verbunden ist und eine zweite darin befindliche Öffnung (28) hat,
**dadurch gekennzeichnet, dass** das erste und das zweite Element (20, 22) außer Flucht miteinander sind, wodurch das erste und das zweite Element (20, 22) und die erste und die zweite Öffnung (26, 28) als Ventil wirken, so dass, wenn eine Hülle (16) in die erste und die zweite Öffnung (26, 28) geschoben wird und mit der ersten Öffnung (26) in Eingriff gelangt, sich das erste Element (20) relativ zum zweiten Element (22) um das Gelenk (24) biegen kann und, wenn die Hülle (16) nicht mehr geschoben wird, die federnde Verbindung der Elemente (20, 22) durch das Gelenk (24) bewirkt, dass die erste und die zweite Öffnung (26, 28) die Hülle (16) erfassen und die Hülle (16) gegen ein Drehen innerhalb der Öffnungen (26, 28) halten.

2. Vorrichtung nach Anspruch 1, wobei die Anordnung derart ist, dass der Vorgang des Herausziehens der Hülle (16) aus den Öffnungen bewirkt, dass sich das erste und das zweite Element (20, 22) relativ zueinander um das Gelenk (24) biegen, wodurch das Erfassen der Hülle (16) seitens der Elemente verstärkt wird.

3. Vorrichtung nach Anspruch 1 oder Anspruch 2, wobei die erste und die zweite Öffnung (26, 28) konfiguriert sind, eine Hülle (16) einer Nadel (15) mit einer Querschnittsweite im Bereich von 4 mm² bis 10 mm², z.B. 4 bis 6 mm², so aufzunehmen, dass sie engsitzend sowohl in die erste als auch in die zweite Öffnung (26, 28) passt und die Öffnungen gegebenenfalls so konfiguriert sind, dass sie eine Hülle (16) einer Nadel engsitzend aufnehmen, die eine Querschnittsbreite irgendwo im Bereich von 4 mm bis 10 mm, z.B. im Bereich von 4 bis 6 mm, hat.

4. Vorrichtung nach einem der Ansprüche 1 bis 3, wobei, wenn sie zur Aufnahme der Hülle (16) benützt wird, imaginäre Linien, die orthogonal durch die Zentren der ersten und der zweiten Öffnung (26, 28) hindurchgehen, in einem Winkel α von 170° bis 110° zueinander liegen.

5. Vorrichtung nach einem der Ansprüche 1 bis 4, wobei, wenn sie zur Aufnahme der Hülle (16) benützt wird und das Gelenk (24) nicht gebogen ist, das erste Element (20) in einem Winkel von 10° bis 60° zum zweiten Element liegt.

6. Vorrichtung nach einem der Ansprüche 1 bis 5, welche mindestens ein weiteres Element inkludiert, das eine Öffnung darin hat und das federnd mit einem vorhergehenden Element durch ein Gelenk (24) verbunden ist, und wobei die Öffnung in jedem weiteren Element außer Flucht mit der Öffnung im vorhergehenden Element ist.

7. Vorrichtung nach einem der Ansprüche 1 bis 6, hergestellt aus Papierzellstoff/geformter Faser, Metall oder Kunststoffmaterial.

8. Vorrichtung nach einem der Ansprüche 1 bis 7, wobei jede der Öffnungen (26, 28) mindestens eine federnde Klappe (27) aufweist, die in die Öffnung hineinragt und an einer in die Öffnung eingeführten Hülle (16) angreift, um einen engsitzenden Eingriff der Hülle (16) mit der Öffnung vorzusehen.

9. Vorrichtung, die zum Erfassen einer medizinischen Nadel konfiguiert ist, so dass die Nadel (15) in einem sicheren Zustand gehalten werden kann, welche Vorrichtung aufweist:
ein erstes Element (20) mit einem ersten Bereich, den eine Nadel (15) leichter durchdringen kann als den Rest des ersten Elements (20), und
ein zweites Element (22), das mit dem ersten Element (20) durch ein Gelenk (24) federnd verbunden ist und einen zweiten Bereich aufweist, den eine Nadel (15) leichter durchdringen kann als den Rest des zweiten Elements,
**dadurch gekennzeichnet, dass** die Anordnung derart ist, dass, wenn eine Nadel (15) die Bereiche im ersten und im zweiten Element (20, 22) durchdrungen hat, so dass die Nadel (15) innerhalb von Löchern in den Bereichen des ersten und des zweiten Elements (20, 22) gehalten ist, der Vorgang des Versuchs, die Nadel (15) aus dem ersten und dem zweiten Element (20, 22) zurückzuziehen, bewirkt, dass sich die Elemente um das Gelenk (24) bewegen, so dass die durch das erste und das zweite Element (20, 22) durchgehenden Löcher aus dem Fluchten gebracht werden, wodurch bewirkt wird, dass die Nadel (15) durch ihren Eingriff mit dem ersten und dem zweiten Element (20, 22) ergriffen wird.

10. Vorrichtung nach Anspruch 9, wobei die Bereiche aus einem weicheren und/oder dünneren Material als der Rest der entsprechenden Elemente gebildet sind.

11. Vorrichtung aus einem nachgiebigen, federnden Material, z.B. Papierzellstoff oder Kunststoffmaterial, welche zum Erfassen der Hülle (16) einer medizinischen Nadel (15) konfiguriert ist, so dass die Nadel (15) mit einer Hand daraus zurückgezogen werden kann, und zum Halten der Hülle (16), während die Nadel (15) in Verwendung ist, welche Vorrichtung mindestens zwei federnd miteinander verbundene Elemente und eine zwischen den Elementen gebildete Öffnung aufweist, in welche die Hülle (16) eingeführt werden kann,
wobei die mindestens zwei Elemente (32,52) und die mindestens eine Öffnung als Ventil wirken, welches ermöglicht, dass die Hülle (16) geschoben wird, um sie in die mindestens eine Öffnung einzuführen, welches jedoch die Hülle (16) erfasst, wenn die Hülle (16) aus der Öffnung herausgezogen wird, wobei die federnde Verbindung zwischen den Elementen konfiguriert ist, eine Hülle (16) gegen ein Drehen innerhalb der Öffnung zu halten,
wobei die Elemente in Bezug aufeinander in einer Konfiguration so abgewinkelt sind, dass eine Hülle (16), wenn sie in einer Richtung durch die Öffnung bewegt wird, mit den an die Öffnung angrenzenden Elementen in Eingriff gelangt und eine weitere Bewegung in dieser Richtung dazu neigt, die Öffnung zu erweitern, wodurch es der Hülle (16) ermöglicht wird, sich weiter in dieser Richtung zu bewegen, und so, dass eine Hülle (16), wenn sie in der entgegengesetzten Richtung durch die Öffnung bewegt wird, mit den an die Öffnung angrenzenden Elementen in Eingriff gelangt und eine weitere Bewegung in dieser Richtung dazu neigt, die Öffnung zusammenzuziehen, wodurch sie der sich in dieser Richtung bewegenden Hülle (16) widersteht,
**dadurch gekennzeichnet, dass** die Elemente Teil einer Konstruktion (30, 50) sind, die sich in der Einführungsrichtung der Hülle (16) erstreckt, so dass die Konstruktion zusammengedrückt werden kann, um die Elemente mit einer darin eingeführten Hülle (16) zum Fluchten zu bringen, um zu ermöglichen, dass die Hülle (16) von zwischen den Elementen herausgezogen wird.

12. Vorrichtung nach Anspruch 11, wobei die Elemente (32, 52), die in Bezug aufeinander abgewinkelt sind, einen Teil einer Rundung oder eine "V"-Form bilden.

13. Vorrichtung nach einem vorhergehenden Anspruch, wobei die Elemente (32, 52) integral, z.B. in einem einzigen Formteil, geformt sind.

14. Halter, z.B. Tablett, zum Halten einer medizinischen Nadel (15), einer über der Nadel aufgesetzten Hülle (16), und einer Spritze, wobei der Halter eine Vorrichtung nach einem der Ansprüche 1 bis 13 aufweist, wobei die Vorrichtung:
gegebenenfalls vom Rest des Halters trennbar ist, beispielsweise mit dem Rest des Halters durch eine Schwächungslinie verbunden ist, oder ein separates Stück ist, welches mit dem Halter verbunden ist und entfernt werden kann, und
gegebenenfalls eine flache Oberfläche oben am Halter gegenüber jener Stelle der Vorrichtung aufweist, die als Handgelenkstütze fungieren kann, um den Halter ruhig zu halten, während eine Nadel umhüllt wird, welche Handgelenkstütze gegebenenfalls über den Halter gebogen sein kann, um einen Daumenschutz oder eine Abdeckung für den Halter vorzusehen.

15. Verfahren zum Entfernen einer Hülle (16) von einer medizinischen Nadel (15) unter Verwendung einer Vorrichtung nach einem der Ansprüche 1 bis 8, welches Verfahren umfasst:
a. Bewegen der Hülle (16) in die Öffnungen im ersten und im zweiten Element (20, 22) in eine erste Richtung, wobei die Hülle (16) gegebenenfalls engsitzend in die Öffnungen im ersten und im zweiten Element (20, 22) passt;
b. Schieben der Hülle (16) in die Öffnungen zum Eingriff mit den an die Öffnungen grenzenden Elementen;
c. Freigabe der Schiebekraft, um es den Elementen zu ermöglichen, die Hülle (16) zu erfassen;
d. Zurückziehen der medizinischen Nadel aus der erfassten Hülle (16), z.B. durch Ziehen der medizinischen Nadel in die zur ersten Richtung entgegengesetzte Richtung, was gegebenenfalls bewirkt, dass sich das erste und das zweite Element (20, 22) um das Gelenk (24) bewegen, was die die Hülle (16) erfassende Kraft steigert, bis die Nadel aus der Hülle (16) gezogen ist;
e. Wiedereinführen der Nadel in die von der Vorrichtung gehaltene Hülle (16), und
f. Drehen und Ziehen an einer mit der Nadel verbundenen Spritze, um die Spritze von der Nadel zu trennen,
und gegebenenfalls:
g. Halten des ersten und/oder des zweiten Elements (22) an Ort und Stelle, um die Relativbewegung der beiden Elemente um das Gelenk (24) zu verhindern, wodurch ermöglicht wird, dass die Hülle (16) aus der Vorrichtung zurückgezogen wird.

## Revendications

1. Dispositif qui est configuré pour saisir la gaine (16) d'une aiguille médicale de sorte que l'aiguille (15) peut en être rétractée d'une main et pour maintenir la gaine pendant que l'aiguille (15) est en cours d'utilisation, lequel dispositif comprend :
un premier élément (20) ayant une première ouverture (26) dedans et un deuxième élément (22) joint élastiquement au premier élément (20) par une articulation (24) et ayant une deuxième ouverture (28) dedans, **caractérisé en ce que** les premier et deuxième éléments (20, 22) sont désalignés l'un par rapport à l'autre, moyennant quoi lesdits premier et deuxième éléments (20, 22) et les première et deuxième ouvertures (26, 28) agissent en tant que soupape de sorte que, lorsqu'une gaine (16) est poussée dans les première et deuxième ouvertures (26, 28) et vient en prise avec le premier orifice (26), le premier élément (20) peut fléchir par rapport au deuxième élément (22) autour de l'articulation (24) et lorsque la gaine (16) n'est plus poussée, la liaison élastique des éléments (20, 22) par l'articulation (24) fait en sorte que les première et deuxième ouvertures (26, 28) saisissent la gaine (16) et empêchent la gaine (16) de tourner au sein des ouvertures (26, 28).

2. Dispositif selon la revendication 1, dans lequel l'agencement est tel que l'action de traction de la gaine (16) hors des ouvertures fait en sorte que les premier et deuxième éléments (20, 22) fléchissent les uns par rapport aux autres autour de l'articulation (24), en augmentant de ce fait la prise des éléments sur la gaine (16).

3. Dispositif selon la revendication 1 ou la revendication 2, dans lequel les première et deuxième ouvertures (26, 28) sont configurées pour recevoir une gaine (16) d'une aiguille (15) ayant une largeur en coupe dans l'intervalle allant de 4 mm à 10 mm carrés, par exemple 4 à 6 mm carrés, de telle sorte qu'elle s'ajuste confortablement dans l'une et l'autre des première et deuxième ouvertures (26, 28) et éventuellement les ouvertures sont configurées pour recevoir confortablement une gaine (16) d'une aiguille qui a une largeur en coupe n'importe où dans l'intervalle allant de 4 mm à 10 mm, par exemple n'importe où dans l'intervalle allant de 4 à 6 mm.

4. Dispositif selon l'une quelconque des revendications 1 à 3, dans lequel, lors d'un déploiement pour recevoir la gaine (16), des lignes théoriques passant orthogonalement à travers les centres des première et deuxième ouvertures (26, 28) se trouvent à un angle a de 170° à 110° les unes par rapport aux autres.

5. Dispositif selon l'une quelconque des revendications 1 à 4, dans lequel, lors d'un déploiement pour recevoir la gaine (16) et lorsque l'articulation (24) n'est pas fléchie, le premier élément (20) se trouve à un angle de 10° à 60° par rapport au deuxième élément.

6. Dispositif selon l'une quelconque des revendications 1 à 5, qui comprend au moins un élément supplémentaire qui a une ouverture dedans et qui est relié élastiquement à un élément précédent par une articulation (24), et dans lequel l'ouverture dans chaque élément supplémentaire se trouve désalignée par rapport à l'ouverture dans l'élément précédent.

7. Dispositif selon l'une quelconque des revendications 1 à 6, constitué d'un matériau de fibre de pâte à papier/moulée, de métal ou de plastique.

8. Dispositif selon l'une quelconque des revendications 1 à 7, dans lequel chacune des ouvertures (26, 28) comprend au moins un volet élastique (27) qui s'étend dans l'ouverture et vient en prise avec une gaine (16) insérée dans l'ouverture pour fournir une mise en prise confortable de la gaine (16) avec l'ouverture.

9. Dispositif qui est configuré pour saisir une aiguille médicale de sorte que l'aiguille (15) peut être maintenue dans une condition sûre, lequel dispositif comprend :
un premier élément (20) ayant une première région qu'une aiguille (15) peut pénétrer plus facilement que le résidu du premier élément (20) et
un deuxième élément (22) joint élastiquement au premier élément (20) par une articulation (24) et ayant une deuxième région qu'une aiguille (15) peut pénétrer plus facilement que le reste du deuxième élément, **caractérisé en ce que** l'agencement est tel que, lorsqu'une aiguille (15) a pénétré les régions dans les premier et deuxième éléments (20, 22) de telle sorte que l'aiguille (15) est maintenue au sein des trous dans lesdites régions des premier et deuxième éléments (20, 22), l'action consistant à essayer de retirer l'aiguille (15) des premier et deuxième éléments (20, 22) fait en sorte que les éléments se déplacent autour de l'articulation (24) de sorte que les trous à travers les premier et deuxième éléments (20, 22) sont amenés en désalignement, en faisant en sorte de ce fait que l'aiguille (15) est saisie par sa mise en prise avec les premier et deuxième éléments (20, 22).

10. Dispositif selon la revendication 9, dans lequel lesdites régions sont formées d'un matériau plus mou et/ou plus mince que le reste des éléments respectifs.

11. Dispositif constitué d'un matériau élastique conforme, par exemple un matériau de pâte à papier ou en plastique qui est configuré pour saisir la gaine (16) d'une aiguille médicale (15) de sorte que l'aiguille (15) peut en être rétractée d'une main et pour maintenir la gaine (16) pendant que l'aiguille (15) est en cours d'utilisation, lequel dispositif comprend au moins deux éléments reliés élastiquement ensemble et une ouverture formée entre les éléments dans laquelle la gaine (16) peut être insérée,
lesdits au moins deux éléments (32, 52) et l'au moins une ouverture agissant en tant que soupape qui permet à la gaine (16) d'être poussée pour l'insérer dans l'au moins une ouverture, mais saisit la gaine (16) si la gaine (16) est retirée de l'ouverture, dans lequel la liaison élastique entre les éléments est configurée pour empêcher une gaine (16) de tourner au sein de l'ouverture,
dans lequel les éléments sont inclinés les uns par rapport aux autres dans une configuration telle qu'une gaine (16) lorsqu'elle est déplacée dans une direction à travers l'ouverture vient en prise avec les éléments bordant l'ouverture et un mouvement ultérieur dans cette direction a tendance à dilater l'ouverture, permettant de ce fait à la gaine (16) de se déplacer plus loin dans cette direction, et de telle sorte qu'une gaine (16) lorsqu'elle est déplacée dans la direction opposée à travers l'ouverture vient en prise avec les éléments bordant l'ouverture et un mouvement ultérieur dans cette direction a tendance à contracter l'ouverture, empêchant de ce fait la gaine (16) de se déplacer dans cette direction
**caractérisé en ce que** les éléments font partie d'une structure (30, 50) qui s'étend dans la direction d'insertion de la gaine (16) de telle sorte que la structure peut être comprimée pour aligner les éléments avec une gaine insérée (16) pour permettre à la gaine (16) d'être retirée d'entre les éléments.

12. Dispositif selon la revendication 11, dans lequel les éléments (32, 52) qui sont inclinés les uns par rapport aux autres forment une partie d'un dôme ou d'une « forme en V ».

13. Dispositif selon l'une quelconque des revendications précédentes, dans lequel les éléments (32, 52) sont formés en une seule pièce, par exemple dans un seul moulage.

14. Support, par exemple un plateau, pour contenir une aiguille médicale (15), une gaine (16) ajustée sur l'aiguille et une seringue, lequel support comprend un dispositif selon l'une quelconque des revendications 1 à 13, ledit dispositif :
étant éventuellement séparable du reste du support, par exemple il est joint au reste du support par une ligne de faiblesse, ou est une pièce indépendante qui est reliée au support et peut être enlevée et comprenant éventuellement une surface plate sur la partie supérieure du support opposée à l'emplacement du dispositif qui peut agir en tant que repose-poignet pour maintenir le support tout en gainant une aiguille, lequel repose-poignet peut éventuellement être replié sur le support pour fournir une protection pour le pouce ou un couvercle pour le support.

15. Procédé de retrait d'une gaine (16) d'une aiguille médicale (15) en utilisant un dispositif selon l'une quelconque des revendications 1 à 8, lequel procédé comprend :
a. le déplacement de la gaine (16) dans les ouvertures dans les premier et deuxième éléments (20, 22) dans une première direction, la gaine (16) s'ajustant éventuellement confortablement dans les ouvertures dans les premier et deuxième éléments (20, 22) ;
b. la poussée de la gaine (16) dans les ouvertures pour amener en prise les éléments bordant les ouvertures ;
c. le relâchement de la force de poussée pour permettre aux éléments de saisir la gaine (16) ;
d. la rétraction de l'aiguille médicale de la gaine saisie (16), par exemple en tirant l'aiguille médicale dans la direction opposée à la première direction, qui fait éventuellement en sorte que les premier et deuxième éléments (20, 22) se déplacent autour de l'articulation (24), en augmentant ainsi la force saisissant la gaine (16) jusqu'à ce que l'aiguille sorte de la gaine (16) ;
e. la réinsertion de l'aiguille dans la gaine (16) maintenue par le dispositif, et
f. la torsion et la traction d'une seringue reliée à l'aiguille pour déconnecter la seringue de l'aiguille
et éventuellement :
g. le maintien des premier et/ou deuxième éléments (22) en place pour empêcher le mouvement relatif des deux éléments autour de l'articulation (24), permettant de ce fait à la gaine (16) d'être rétractée du dispositif.
